# EUROPEAN PATENT SPECIFICATION

(11) **EP 3 897 648 B1**
(45) Date of publication and mention of the grant of the patent: **23.08.2023**
(21) Application number: 19836558.7
(22) Date of filing: 18.12.2019
(51) Int. Cl.: A61K 31/506, A61K 31/635, A61K 39/395, A61K 45/06, A61P 35/02, C07K 16/28

(54) **EXTENDED LOW DOSE REGIMENS FOR MDM2 INHIBITORS**
ERWEITERTE NIEDRIGDOSIERUNGSSCHEMATA FÜR MDM2-INHIBITOREN
RÉGIMES À FAIBLE DOSE ÉTENDUE POUR INHIBITEURS DE MDM2

(30) Priority: 20.12.2018 US 201862782727 P; 20.12.2018 US 201862782730 P; 20.12.2018 US 201862782735 P
(43) Date of publication of application: 27.10.2021
(73) Proprietor: Novartis AG, 4056 Basel (CH)
(72) Inventor: GUERREIRO, Nelson, 4002 Basel (CH); JULLION, Astrid, 4002 Basel (CH); MEILLE, Christophe, 4002 Basel (CH); FABRE, Claire, 4002 Basel (CH)
(74) Representative: Ridout, Joseph
(86) International application number: PCT/IB2019/061012
(87) International publication number: WO 2020/128892

(56) References cited:
- WO-A1-2018/092020
- WO-A1-2018/178925
- HYMAN D ET AL: "Dose- and regimen-finding phase I study of NVP-HDM201 in patients (pts) with TP53 wild-type (wt) advanced tumors", EUROPEAN JOURNAL OF CANCER, vol. 69, 1 December 2016 (2016-12-01), XP029843847, ISSN: 0959-8049, DOI: 10.1016/S0959-8049(16)32982-3
- D M Hyman ET AL: "Dose- and Regimen-finding Phase I Study of NVP-HDM201 in Patients With TP53 Wild-type Advanced Tumors (Poster presented at EORTC-NCI-AACR Munic, Germany, 29.11.2016-2.12.2016)", , 2 December 2016 (2016-12-02), XP55486600, Retrieved from the Internet: URL:http://www.poster-submission.com/ena20 16/visitors/eposter/33139 [retrieved on 2018-06-21]
- B. HIGGINS ET AL: "Preclinical Optimization of MDM2 Antagonist Scheduling for Cancer Treatment by Using a Model-Based Approach", CLINICAL CANCER RESEARCH, vol. 20, no. 14, 15 July 2014 (2014-07-15) , pages 3742-3752, XP55205610, ISSN: 1078-0432, DOI: 10.1158/1078-0432.CCR-14-0460
- JAN DAVIDSON-MONCADA ET AL: "Dissecting the Immune Landscape of Acute Myeloid Leukemia", BIOMEDICINES, vol. 6, no. 4, 25 November 2018 (2018-11-25), page 110, XP55675494, DOI: 10.3390/biomedicines6040110
- Anonymous: "History of Changes for Study: NCT03940352 HDM201 in Combination With MBG453 or Venetoclax in Patients With Acute Myeloid Leukemia (AML) or High-risk M Syndrome (MDS)", ClinicalTrials.gov archive, 15 October 2019 (2019-10-15), pages 1-5, XP55675380, Retrieved from the Internet: URL:https://clinicaltrials.gov/ct2/history /NCT03940352?V_4=View#StudyPageTop [retrieved on 2020-03-10]

## Description

### FIELD OF THE INVENTION

The present invention relates to the HDM2-p53 interaction inhibitor (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable salt, solvate, hydrate, complex or co-crystal thereof for use in the treatment of hematological tumors, wherein the drug is administered on each of the first 3 to 7 days of a 28 days treatment cycle, wherein the treatment is composed of at least three 28 days treatment cycles, wherein the daily drug dose for the first and second treatment cycles, which are induction cycles is from 50 mg to 100 mg and the daily drug dose for the third and any following treatment cycles, which are consolidation cycles is from 10 mg to 45 mg.

### BACKGROUND OF THE INVENTION

p53 is induced and activated by a number of potentially tumorigenic processes - including aberrant growth signals, DNA damage, ultraviolet light, and protein kinase inhibitors (Millard M, et al. Curr Pharm Design 2011;17:536-559) - and regulates genes controlling cell growth arrest, DNA repair, apoptosis, and angiogenesis (Bullock AN & Fersht AR. Nat Rev Cancer 2001;1:68-76; Vogelstein B, et al. Nature Education 2010;3(9):6).

Human Double Minute-2 (HDM2) is one of the most important regulators of p53. It binds directly to p53, inhibiting its transactivation, and subsequently directing it towards cytoplasmic degradation (Zhang Y, et al. Nucleic Acids Res 2010;38:6544-6554).

p53 is one of the most frequently inactivated proteins in human cancer, either through direct mutation of the *TP53* gene (found in approximately 50% of all human cancers) (Vogelstein, B et al. Nature 2000;408:307-310) or via suppressive mechanisms such as overexpression of HDM2 (Zhao Y, et al. BioDiscovery 2013;8:4)*.*

Potent and selective inhibitors of the HDM2-p53 interaction (also referred to as HDM2 inhibitors or MDM2 inhibitors), e.g. NVP-HDM201 (herein referred to as HDM201), have been shown to restore p53 function in preclinical cell and in vivo models (Holzer P, et al. Poster presented at AACR 2016, Abstract #4855, Holzer P, Chimia 2017, 71(10), 716-721). The HDM2 inhibitor HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one, and methods how to prepare it were disclosed for example in WO2013/111105.

Different dosing regimens were described for HDM2 inhibitors and tested in clinical studies. E.g. US2013/0245089 discloses a method of treating a patient suffering from cancer by administering to the patient 4-{[(2R,3S,4R,5S)-4-(4-Chloro-2-fluoro-phenyl)-3-(3-chloro-2-fluoro-phenyl)-4-cyano-5-(2, 2-dimethyl-propyl)-pyrrolidine-2-carbonyl]-amino}-3-methoxybenzoic acid in an amount of from about 800 to about 3000 mg/day for an administration period of up to about 7 days, on days 1-7, of a 28 days treatment cycle, followed by a rest period of from about 21 to about 23 days.

A paper in Clinical Cancer Research by B. Higgins et al, in May 2014 (Higgins B. et al, Preclinical Optimisation of MDM2 Antagonist Scheduling for Cancer Treatment by Using a Model-Based Approach. Clin Cancer Research 2014; 20:3742-3752,disclosed a 28-day cycle schedule, where RG7388 is administered once weekly three times followed by 13 days of rest (28 days cycle schedule), or where the drug is administered for 5 consecutive days of a 28 days schedule.

Further dosing regimens for HDM2 inhibitors, e.g. intermittent high dose regimens and extended low dose regiments are disclosed in WO 2015/198266, WO 2018/092020, and WO 2018/178925.

However, long term platelet depletion and/or disease resistance limiting drug effect on bone marrow blasts in later treatment cycles is a common challenge in the therapies involving HMD2 inhibitors. Therefore, there remains a need for optimizing dose and regimens of these anti-cancer drugs to minimize the adverse effects.

WO2018/178925 discloses an intermittent dosing regimen of HDM201 for hematological tumors, particularly wherein the drug is administered on each of the first 6 to 8 days of a 28 day treatment cycle, wherein the treatment is composed of at least two 28 day treatment cycles, and wherein the daily drug dose is from 40 mg to 90 mg.

WO2018/092020 discloses an intermittent dosing regimen of HDM201, particularly wherein the drug is administered on two different administration days within a treatment cycle, wherein the first administraton day and the second administration day are interrupted by a short administration-free period, and the second administration day of the first or earlier treatment cycle and the first administration of the followinbg cycle are interrupted by a long administration-free period, wherein the short administration-free period is composed of from 4 to 8 days, and the long administration-free period is composed of from 13 to 27 days, and wherein the treatment is composerd of at least 2 treatment cycles.

Hyman D et al: "Dose and regimen-finding phase I study of NVP-HDM201 in patients (pts) with TP53 wild-type (wt) advanced tumors", "EUROPEAN JOURNAL OF CANCER, vol. 69, XP0298438847, ISSN: 0959-8049, DOI: 10.1016/S0959-8049(16)32982-3 and associated poster presented at EORT-NCI-AACR, Munich, Germany, 29.11.2016-2.12.2026, XP5548660 discusess four oral treatment regimens of HDM201: Regimen 1A , a single dose on day 1 in a 3 week cycle, Regimen 1B, dosing on days 1 and 8 of a 4 week cycle, Regimen 2A, dosing daily for the first two weeks of a four week cycle, and Regimen 2C, dosing daily for the first week of a four week cycle.

### SUMMARY OF THE INVENTION

Any references to methods of treatment in the paragraphs of this description are to be interpreted as references to the compounds, pharmaceutical compositions or medicaments of the present invention for use in a method for treatment of the human (or animal) body by therapy (or for diagnosis).

One of the objectives in the development of an HDM2 inhibitor drug is to find a dosing regimen which allows the administration of a dose which ensures efficacy but at the same time reduces the risk of the occurance of adverse events.

It has been surprisingly found that a new type of dosing regimen is particularly useful for the treatment of hematological tumors with the HDM2 inhibitor HDM201.

Specifically, the present invention provides the following aspects, advantageous features and specific embodiments, respectively alone or in combination, as listed in the following items:
The HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable non-covalent derivative (including salt, solvate, hydrate, complex, co-crystal) thereof,
for use in the treatment of hematological tumors,
wherein the drug is administered on each of the first 3 to 7 days of a 28 days treatment cycle, wherein the treatment is composed of at least three 28 days treatment cycles,
wherein the daily drug dose for the first and second treatment cycles, which are induction cycles is from 50 mg to 100 mg and the daily drug dose for the third and any following treatment cycles which are consolidation cycles is from 10 mg to 45 mg.

This dosing regiment is particularly advantageous for the treatment of leukemias, e.g. AML or MDS and/or when the hematological tumor is TP53 wild-type.

The treatment of the hematological tumors with HDM201 according to the present invention may also comprise one or more further anti-cancer agents (standard-of-care drugs in the field and/or novel anti-cancer agents, including immuno-oncology drugs).

The dosing regimens of the present invention as described herein provide a highly favorable therapeutic index, low incidence of grade 3/4 thrombocytopenia while achieving therapeutically relevant exposures, p53 pathway activation (GDF-15 upregulation), and clinical activity.

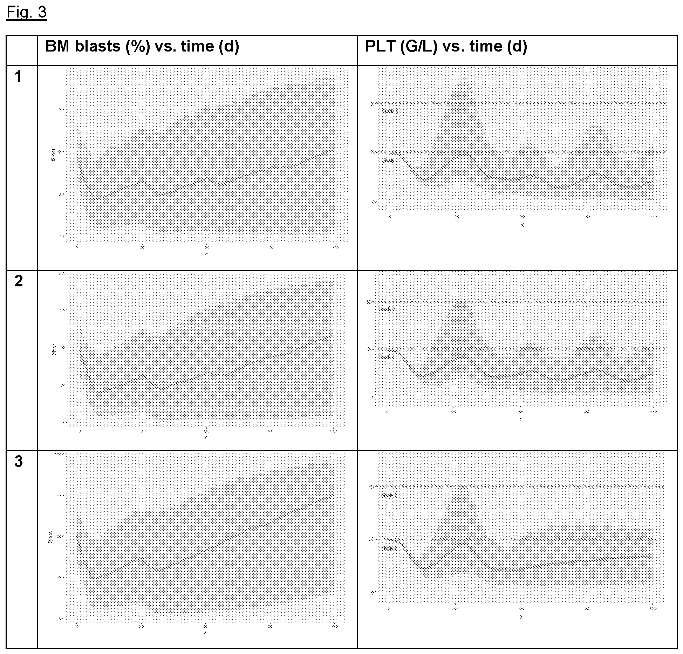

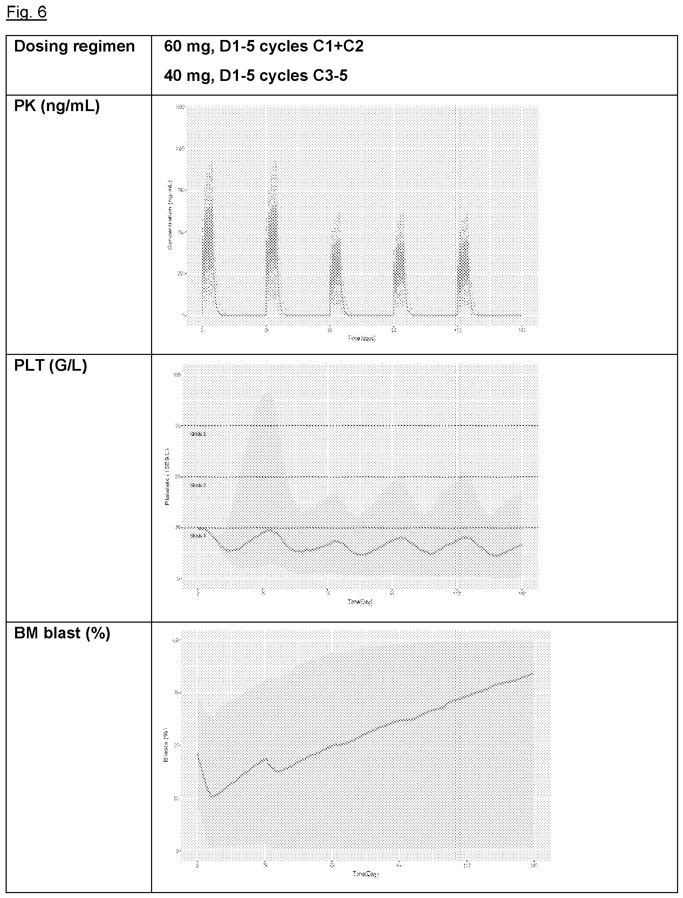

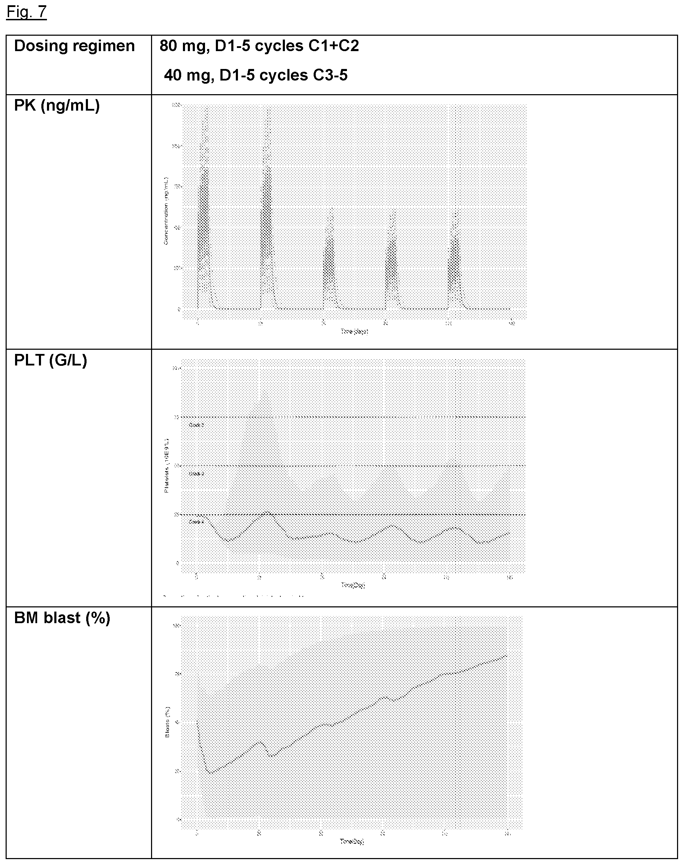

In particular, the dosing regimens of the present invention as described above provide a good bone marrow (BM) blasts response within the first two treatment cycles while managing effectively safety in subsequent treatment cycles (cycles 3 and following), see Figure 3, variant 2 and Figures 6-7.

### BRIEF DESCRIPTION OF THE DRAWINGS

In the following the present invention is described in detail with reference to accompanying figures in which:

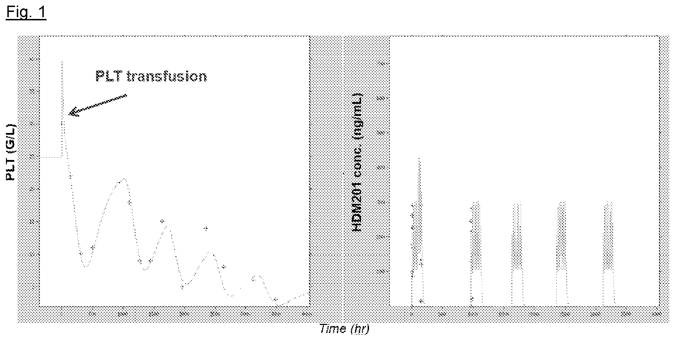

**Figure 1** shows an example of an individual platelet (PLT) profile (Regimen 2C, i.e. d1-7q28d, 45 mg), from clinical study CHDM201X2101.

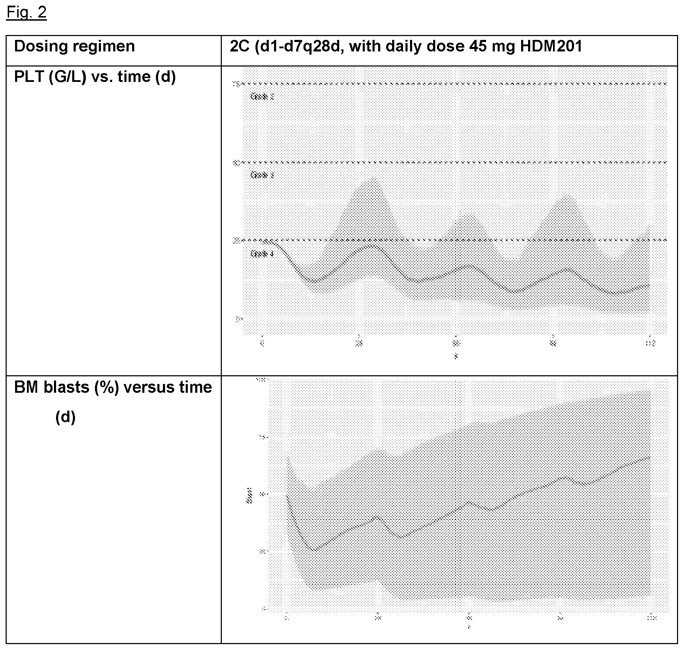

**Figure 2** shows the impact of dosing regimen 2C (d1-d7q28d, with daily dose 45 mg HDM201) on PLT profile is limited with no recovery. Long-term platelet depletion, PLT (G/L) versus time(d), Median and interquartile range. Figure 2 further shows the impact of dosing regimen on blast kinetics: regimen 2C with 45 mg daily dose HDM201 achieves good BM blasts depletion. Early and low nadir. BM blasts (%) versus time (d).
**Figure 3** shows the simulated profile for regiment 2C variants 1, 2, and 3. Variant 1: 60mg (4 cycles); Variant 2: 60mg (2 cycles) → 30mg (2 cycles); Variant 3: 60mg (2 cycles) → 0. Variants 2-3 provide dose(s) to maximize BM blasts response within first 2 cycles, while managing safety in subsequent cycles (cycles 3 and 4).

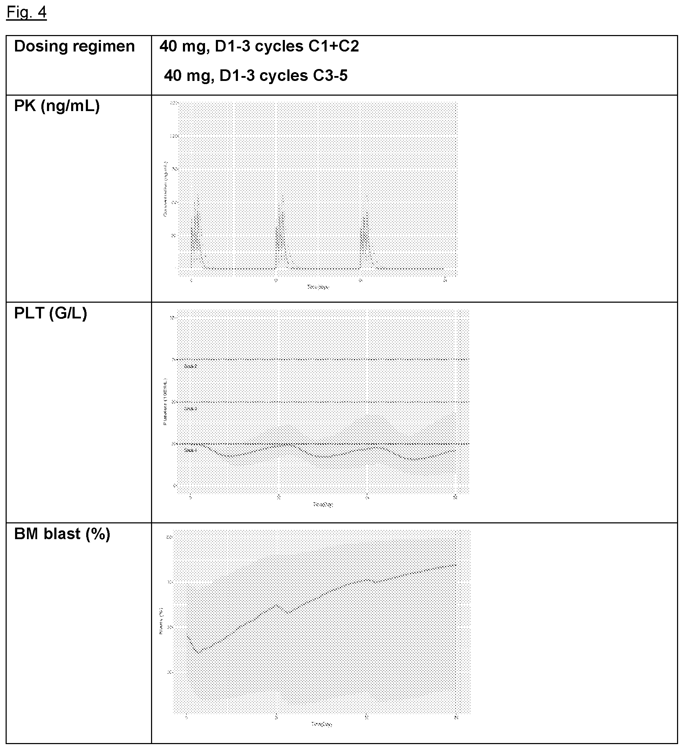

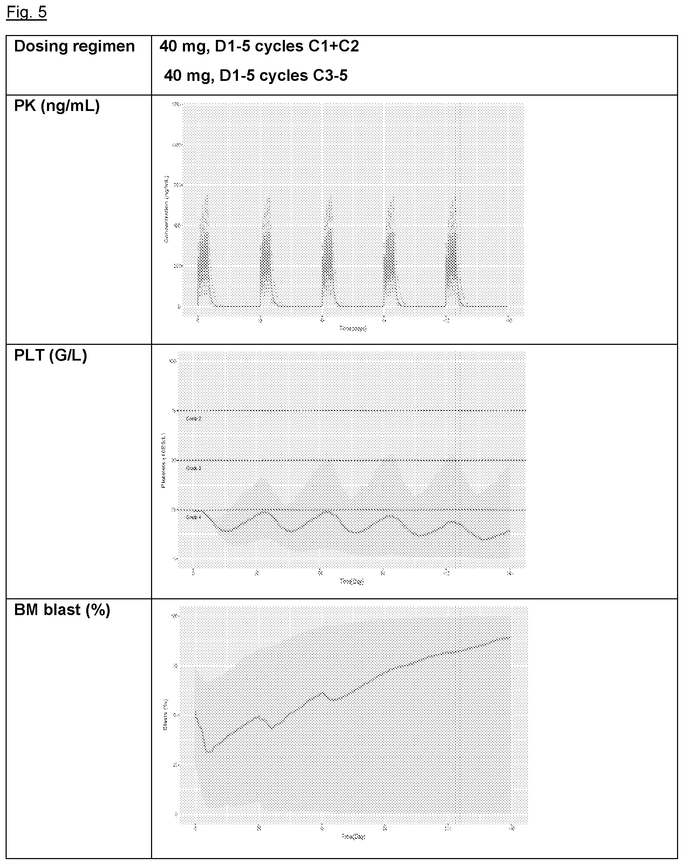

**Figures 4-7** shows the simulation of platelet (PLT) and bone marrow (BM) blast metrics from HDM201X2101 dose(s) to maximize BM blasts response within first 2 cycles, while managing safety in subsequent cycles (cycles 3-5)
**Figure 8** shows the ramp-up (RU) of venetoclax during cycle 1 (treatment arm 2: HDM201+venetoclax)

### DETAILED DESCRIPTION OF THE INVENTION

Herein after, the present invention is described in further detail and is exemplified.

### Definitions

Additional terms are defined below and throughout the application.

As used herein, the articles "a" and "an" refer to one or to more than one (e.g., to at least one) of the grammatical object of the article.

The term "or" is used herein to mean, and is used interchangeably with, the term "and/or," unless context clearly indicates otherwise.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

By "a combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapeutic protocol can be administered in any order. In general, each agent will be administered at a dose and/or on a time schedule determined for that agent. In will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually.

The term "HDM2-p53 interaction inhibitor" or in short "HDM2 inhibitor" is also referred to as "HDM2i", "Hdm2i", "MDM2 inhibitor", "MDM2i", "Mdm2i", denotes herein any compound inhibiting the HDM-2/p53 or HDM-4/p53 interaction with an IC₅₀ of less than 10 µM, preferably less than 1 µM, preferably in the range of nM, measured by a Time Resolved Fluorescence Energy Transfer (TR-FRET) Assay. The inhibition of p53-Hdm2 and p53-Hdm4 interactions is measured by time resolved fluorescence energy transfer (TR-FRET). Fluorescence energy transfer (or Foerster resonance energy transfer) describes an energy transfer between donor and acceptor 5 fluorescent molecules. For this assay, MDM2 protein (amino acids 2-188) and MDM4 protein (amino acids 2-185), tagged with a C-terminal Biotin moiety, are used in combination with a Europium labeled streptavidin (Perkin Elmer, Inc., Waltham, MA, USA) serving as the donor fluorophore. The p53 derived, Cy5 labeled peptide Cy5- TFSDLWKLL (p53 aa18-26) is the energy acceptor. Upon excitation of the donor 10 molecule at 340nm, binding interaction between MDM2 or MDM4 and the p53 peptide induces energy transfer and enhanced response at the acceptor emission wavelength at 665nm. Disruption of the formation of the p53-MDM2 or p53-MDM4 complex due to an inhibitor molecule binding to the p53 binding site of MDM2 or MDM4 results in increased donor emission at 615nm. The ratiometric FRET assay readout is calculated from the 15 raw data of the two distinct fluorescence signals measured in time resolved mode (countrate 665nm/countrate 615nm × 1000). The assay can be performed according to the following procedure: The test is performed in white 1536w microtiterplates (Greiner Bio-One GmbH, Frickenhausen, Germany) in a total volume of 3.1µl by combining 100nl of compounds diluted in 90% DMSO/10% H2O (3.2% final DMSO concentration) with 2µl Europium 20 labeled streptavidin (final concentration 2.5nM) in reaction buffer (PBS, 125mM NaCl, 0.001% Novexin (consists of carbohydrate polymers (Novexin polymers), designed to increase the solubility and stability of proteins; Novexin Ltd., ambridgeshire, United Kingdom), Gelatin 0.01%, 0.2% Pluronic (block copolymer from ethylenoxide and propyleneoxide, BASF, Ludwigshafen, Germany), 1 mM DTT), followed by the addition of 0.5µl MDM2-Bio or MDM4-Bio diluted in assay buffer (final concentration 10nM). Allow the solution to pre-incubate for 15 minutes at room temperature, followed by addition of 0.5µl Cy5-p53 peptide in assay buffer (final concentration 20nM). Incubate at room temperature for 10 minutes prior to reading the plate. For measurement of samples, an Analyst GT multimode microplate reader (Molecular Devices) with the following settings 30 is used: Dichroic mirror 380nm, Excitation 330nm, Emission Donor 615nm and Emission Acceptor 665nm. IC50 values are calculated by curve fitting using XLfit. If not specified, reagents are purchased from Sigma Chemical Co, St. Louis, MO, USA.

The HDM2 inhibitor in accordance with this invention is HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one.

HDM201 may be present as free molecule or in any other non-covalent derivative, including salt, solvate, hydrate, complex, co-crystal or mixtures thereof. HDM201 may be present as acid derivative. The acid derivative may be a salt formed of HDM201 with the acid, or a HDM201 acid complex, or as HDM201 acid co-crystal. Preferably HDM201 is present as co-crystal. Preferably the acid is succinic acid. Most preferably, HDM201 is present as succinic acid co-crystal. Non-covalent derivatives of HDM201 are described in WO2013/111105.

In preferred embodiments, HDM201 is referred to as:
Succinic acid - (6S)-5-(5-chloro-1-methyl-2-oxo-1,2-dihydropyridin-3-yl)-6-(4-chlorophenyl)-2-(2,4 dimethoxypyrimidin-5-yl)-1-isopropyl-5,6-dihydropyrrolo[3,4-d]imidazol-4(1H)-one (1:1).

When referring to a dose amount of HDM201 herein, e.g. in mg (milligram), it is meant to be the amount of HDM201 as free base, in contrast to the salt, solvate, complex, or co-crystal.

The term "hematological tumor" refers herein to a cancer that begins in blood-forming tissue, such as the bone marrow, or in the cells of the immune system. Examples of hematological tumors are leukemia, lymphoma, and multiple myeloma. They are also often referred to as blood cancer.

Preferred hematological tumors of the present invention are leukemias. More preferably, the hematological tumors are selected from acute myeloid leukemia (AML), myelodysplastic syndrome (MDS), and acute lymphoblastic leukemia (ALL). Even more preferably, the hematological tumor is AML and/or MDS.

Particularly preferred hematological tumors of the present invention are *TP53* wild-type hematological tumor. More preferably, the *TP53* wild-type hematological tumors of the present invention are *TP53* wild-type leukemias. Even more preferably, the *TP53* wild-type hematological tumors are selected from TP53 wild-type acute myeloid leukemia (AML), TP53 wild-type myelodysplastic syndrome (MDS), and *TP53* wild-type acute lymphoblastic leukemia (ALL). Even more preferably, the TP53 wild-type hematological tumor is TP53 wild-type AML and/or MDS.

According to the present invention the drug HDM201 is administered on each of the first 3 to 7 days of a 28 days (4 weeks) treatment cycle, preferably the drug is administered on each of the first 4 to 6 days a 28 days treatment cycle, more preferably on the first 5 days of a 28 days treatment cycle.

"On each of the the first 5 days of a 28 days treatment cycle" means that HDM201 is administered to the patient on day 1 (d1), d2, d3, d4, and d5, followed by a drug-administration-free period (also referred to as drug holiday period or rest period) from day 6 until day 28. On day 29 the next treatment cycle starts which will be the d1 of this next treatment cycle.

Preferably, the drug is administered at approximately the same time each administration day (i.e. d1-d5 of a 28 days cycle). Preferably, the drug is administered once daily (qd) on each administration day. More preferably, the drug is administered in the morning.

Preferably, the drug is administered in the fasted state, i.e. at least 1 hour before or 2 hours after a meal.

Preferably the drug is taken with a glass of water and without chewing the capsules or tablet.

If the patient is assigned to a dose level where multiple capsules/tablets are to be taken, the capsules/tablets should be taken consecutively, within as short an interval as possible, e.g. within 5 min.

Preferably, the drug administration is done by oral delivery, i.e. oral administration, per oral (p.o.).

Preferably the drug is provided in the form of an oral dosage form, more preferably in the form of a solid oral dosage form, e.g. a capsule or a tablet.

When dose ranges are given herein, e.g. "the daily drug dose is from 50 mg to 100 mg", any full mg number of the endpoints and in the between those endpoint shall be meant to be disclosed herewith, e.g. 50 mg, 51 mg, 52 mg, 53 mg, 54 mg, 55 mg, 56 mg, 57 mg, ... 98 mg, 99 mg, 100 mg.

As a further aspect of the present invention there is provided:
The HDM2-p53 interaction inhibitor drug HDM201, or a pharmaceutically acceptable non-covalent derivative thereof, for use in the treatment of hematological tumors according to any one of dosing regimens as described herein, wherein the HDM201 drug is combined with one or more other/further anti-cancer agents, preferably said anti-cancer agent(s) is(are) selected from: immuno-oncological drugs (e.g. PD-1 [e.g. PDR001(Novartis, INN Spartalizumab)], PD-L1, LAG-3, TIM-3 [e.g. MBG453(Novartis)], GTIR, TGF-beta, IL15 inhibitors), FLT3 inhibitors (e.g. gilterinib, quizartinib, midostaurin), BCL2 inhibitors (e.g. navitoclax, venetoclax), other HDM2 inhibitors (e.g. idasanutlin, AMG232, DS-3032B, ALRN6924/ATSP7041), hypomethylating agents (HMA) (e.g. Vidaza [azacytidine, 5-azacytidine], Dacogen [decitabine], guadecitabine), anthracyclines (e.g. idarubicin, daunorubicin, doxorubicin, epirubicin, rubidomycin); anti-CD33 antibodies (e.g. Mylotarg [gemtuzumab], vadastuximab) and other agents (e.g. AraC [cytarabine, aracytine]).

Preferably, the drug HDM201 is combined with one or more therapeutically active agents selected from cytarabine (Ara-C), anthracycline, daunorubicin, idarubicin, rubidomycin, idamycin, midostaurin and azacytidine.

In particular preferred embodiments, the drug HDM201 is combined with a BCL2 inhibitor, preferably venetoclax.

In other particular preferred embodiments, the drug HDM201 is combined with a TIM-3 inhibitor, preferably the TIM-3 inhibitor MBG453(Novartis). TIM-3 inhibitors, e.g. MBG453, are described in WO 2015/117002.

The other/further active agents may be dosed on the same day(s) as HDM201 or on days on which no HDM201 dose is administered.

The second medical uses as described in the embodiments of the present invention may be worded in the following various alternative formats:
The HDM2-p53 interaction inhibitor drug HDM201 or a pharmaceutically acceptable salt, solvate, hydrate, complex, or co-crystal thereof for use in the treatment of hematological tumors,
wherein the drug is administered on each of the first 3 to 7 days of a 28 days treatment cycle,
wherein the treatment is composed of at least three 28 days treatment cycles,
wherein the daily drug dose for the first and second treatment cycles, which are induction cycles is from 50 mg to 100 mg and the daily drug dose for the third and any following treatment cycles, which are consolidation cycles is from 10 mg to 45 mg.

A medicament for the treatment of hematological tumors comprising the HDM2-p53 interaction inhibitor drug HDM201 or a pharmaceutically acceptable salt, solvate, hydrate, complex, or co-crystal thereof,
wherein the drug is administered on each of the first 3 to 7 days of a 28 days treatment cycle,
wherein the treatment is composed of at least three 28 days treatment cycles,
wherein the daily drug dose for the first and second treatment cycles, which are induction cycles is from 50 mg to 100 mg and the daily drug dose for the third and any following treatment cycles, which are consolidation cycles is from 10 mg to 45 mg.

### EXAMPLES

### Example 1: Platelet and BM blasts modeling and new dose regimens

### Platelet model

Based on the population PK/PD data of the clinical study CHDM201X2101, an AML patients platelet model was developed which recognizes that the disease influences the regulation of platelets production. The following graphic elucidates the model.

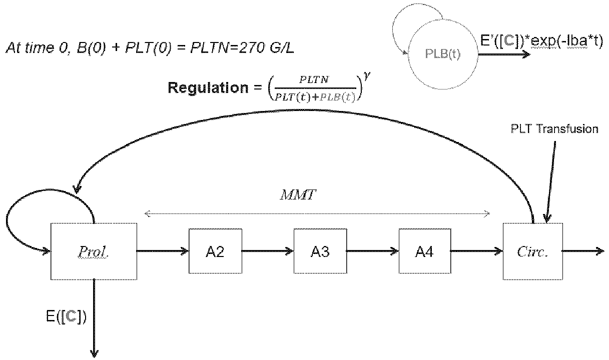

### Bone marrow blasts model

A bone marrow blasts PKPD model were developed which recognizes a delayed effect, a loss of effect with time reproduced by a resistance component, and that a concentrated administration reduces impact of resistance. The following graphic elucidates the model.

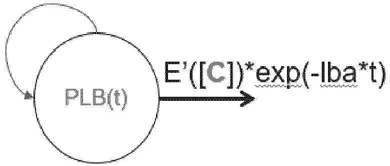

### Derivation of key metrics from simulated platelet and blast profiles

The population PK/PD models of example 1 and 2 were used to simulate PK, platelet and blast profiles over time with inter-individual variability.

The impact of a change in dosing regimen on these profiles were studied.

The simulation design considered: Duration of the cycle, Dose level,, Number of administration, Duration of treatment, Period of induction/consolidation.

The key metrics were: Proportion of patients with platelet counts below/above a given threshold over time, Proportion of patients above PK threshold, Number of days with Blast values below baseline.

The simulations were done using the R (statistical software) with Shiny package.

For model building the PK/PD dataset of CHDM201X2101 were used and an NLME estimation (Monolix 4.3.2) performed. The model structure and the parameter estimates are provided below. This provided inputs for R/shiny. The *mlxR* package were used for simulation of longitudinal data from the MLXTRAN model.

### Model structure

INPUT:
parameter = {r, t1, t2, Tk0, ka, V, Cl, PLTz, MMTP, T12P, sPW, alp, lPW,
kr1, kr1D, EC50, ke0, cfr,h, Sg, HGD, koutg, gdfZ, kinG}

PK:
compartment(cmt=1, amount=Ac)
compartment(cmt=2, amount=P5)
absorption(adm=1, Tlag=t1, Tk0, p=r)
absorption(adm=1, Tlag=t2, ka, p=1-r)
TinfP = 0.5; infusion duration in hours
oral(adm=2, cmt=2, Tk0=TinfP, p=alp)

EQUATION:
odeType = stiff
C = max(1e-16,Ac/V)*1000; convert to ng/mL the concentrations
Cc = C
ke =Cl/V
ddt_Ac = - ke*Ac

ktrP = 4/MMTP
KTR12 = log(2)/T12P
auxF = PLTz/ktrP*KTR12

sfbkP = (PLTz/P5)^(sPW*exp(cfr*E))
lfbkP = (auxF/P1)^lPW

EP1 = kr1*E^h/(E^h+EC50^h) + kr1D*Cc

P1_0 = auxF
P2_0 = auxF
P3_0 = auxF
P4_0 = auxF
P5_0 = PLTz

ddt_P1 = ktrP*(sfbkP-EP1)*P1- ktrP*P1
ddt_P2 = ktrP*lfbkP*sfbkP *P1 - ktrP*P2 ;- EP2*P2
ddt_P3 = ktrP*lfbkP*sfbkP *P2 - ktrP*P3 ;- EP3*P3
ddt_P4 = ktrP*lfbkP*sfbkP *P3 - ktrP*P4 ;- EP4*P4
ddt_P5 = ktrP*lfbkP*sfbkP *P4 - KTR12*P5

ddt_E = ke0*Cc - ke0*E

### Parameter estimates

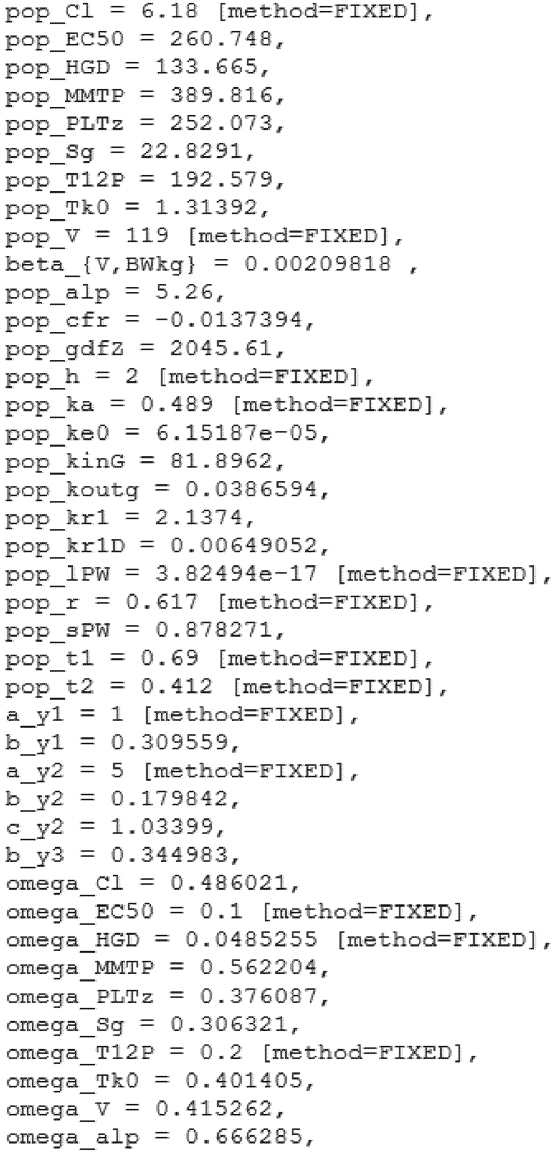

As key findings from PKPD simulations the following was found:
- Long-term platelet depletion and
- Long term treatment (>6 months) is not sustainable without a dose reduction or interruption:
   - Progressive reduction of platelet counts with increasing treatment cycles
   - Disease resistance limiting drug effect on blasts beyond cycle 3 or 4

The simulations support dose and regimen selection for Phase 2 studies in AML.

As a learning from the clinical study CHDM201X2101, the challenges with dosing HDM210 in AML are
- Cumulative platelet toxicity
- Delayed hematopoietic recovery that prevents dosing in consolidation would present a risk to this indication

The present simulation provides a good management of those challenges:
Dose reduction after 1 or 2, preferably 2 cycles of induction.

The simulation was used to support dose escalation strategy in the clinical study HDM201A2101: a new D1-D5 (4 wk cycle) regimen instead of regimen D1-D7 (4 wk cycle) was identified. The following table provides the details of the new dose escalation and new dose regimens.

**Table 1: Simulation of platelet (PLT) and bone marrow (BM) blast metrics from HDM201X2101**

| **Cohort** | Dose Regimen for HDM201 induction Cycles 1+2 | Dose Regimen for HDM201 consolidation Cycles 3-5 | Median % subjects above target [C] from Cycle 1¹ | Median % subjects with at least 1 PLT value above threshold 50G/L² | No. of days with BM blast value below baseline^{2,3} | Median % subjects with PLT decrease from baseline ≥ 50%² | Median % subjects with PLT decrease from baseline ≥ 75%² |
|---|---|---|---|---|---|---|---|
| **-1A** | 60 mg, D1 | 60 mg, D1 | 3 [2.8-3.4] | 3.2 [2.4-3.4] | 7.8 [7.1-8.3] | 29 [27.8-29.8] | 12.6[12.4-13.2] |
| **-1B** | 45 mg, D1-D2 | 45mg, D1-D2 | 15.2 [14-16.2] | 7.8 [7.4-8.2] | 12.1 [11.4-13] | 39.8 [38.8-40.7] | 20.8 [20.4-21.4] |
| **Starting 1** | 40 mg, D1-D3 | 40 mg, D1-D3 | 35 [34-35.6] | 12.2 [11.8-12.6] | 16.8 [15.6-17.3] | 48.6 [48-49.3] | 29.2 [27.9-29.6] |
| **2** | 40 mg, D1-D5 | 40 mg, D1-D5 | 69.4 [69-69.9] | 19.4 [19-19.8] | 38.2 [33.9-41.8] | 63.2 [62.8-63.6] | 41 [40.2-41.6] |
| **3** | 60 mg, D1-D5 | 40 mg, D1-D5 | 89.6 [88.9-90] | 25.2 [24.6-25.8] | 63.1 [58.8-65.9] | 69.9[69.2-70.4] | 48.4 [47.4-49.1] |
| **4** | 80 mg, D1-D5 | 40 mg, D1-D5 | 96.8 [96.4-97.2] | 29.4 [28.2-30] | 82 [77.8-86.2] | 76.4 [74.9-78.8] | 57.4 [56.1-59.7] |
| Note: Metric values represent the Median (2.5%-97.5% percentiles) of 100 repeated simulations performed on 500 subjects. | | | | | | | |
| ¹ average tumor stasis concentration derived from tumor growth inhibition (PK/PD) modeling in xenograft rat model. | | | | | | | |
| ² metric value calculated from Day 1 to Day 140. | | | | | | | |
| ³ subjects with no observed blast reduction from baseline were excluded from the metric derivation. | | | | | | | |

### Example 2: Clinical trials

The advantageous effect of the new dosing regimens as described herein can be demonstrated by clinical trials as outlined in the following.

### 1. Clinical Trial CHDM201A2101

A phase I/II multi-center study of HDM201 added to chemotherapy in adult subjects with relapsed/refractory (R/R) or newly diagnosed acute myeloid leukemia (AML).

### Purpose

HDM201 is a novel investigational agent with promising single-agent activity in AML patients. Study CHDM201X2101 investigated several dose regimens of single agent HDM201 in R/R AML subjects. CR/CRi rate of 24% was reported in subjects with R/R AML who received oral HDM201 at a dose of 45 mg from D1 to D7 every 4 weeks (Q4W) (Regimen 2C), which was the dose assessed as recommended dose for expansion (RDE). The response was rapidly seen within the first two cycles of HDM201, and duration of responses was up to 152 days with HDM201. Most frequent AEs are hematological (about 20% to 50% Grade 3 or 4 neutropenia and thrombocytopenia), gastro-intestinal (only low grade), and tumor lysis syndrome (TLS) (about 10% Grade 3 or 4) in subjects treated at RDE (Regimen 2C: 45 mg D1 to D7 Q4W).

This study is a dose escalation (Part 1) and expansion study (Part 2) conducted in subjects with newly diagnosed untreated (1L AML) or relapsed/ refractory AML (R/R AML), evaluating the safety, tolerability and preliminary activity of HDM201, when combined with chemotherapy. The main objective of Part 1 is to determine RDE (recommended dose for expansion) in 1L and R/R AML subjects. The main objective of Part 2 is to determine the Recommended Phase 3 Dose (RP3D) for HDM201 when combined with chemotherapy in 1L AML subjects without documented FLT3 mutation and R/R AML subjects irrespective of FLT3 status. Another objective of this part is to assess overall safety and tolerability of HDM201 in combination with midostaurin and chemotherapy in 1L AML subjects with documented FLT3 mutation, or in combination with liposomal cytarabine/daunorubicin in secondary AML subjects. Determination of RP3D will be based on an assessment of safety and tolerability of HDM201 in combination with chemotherapy and additionally on the CR/CRi with adequate blood count recovery (ABCR) rate. After RP3D in R/R AML subjects is determined, DDI assessments (Part 3) will be conducted in the R/R AML subjects to evaluate the pharmacokinetic characteristics of HDM201 in the presence of posaconazole (strong CYP3A4 inhibitor), and the potential effect of HDM201 on the pharmacokinetics of midazolam (sensitive CYP3A4 substrate).

### Objectives and endpoints

The objectives and endpoints are presented by part in the following tables below.

**Table 0-1 Part 1 - Dose Escalation: Objectives and Endpoints**

| **Objective(s)** | **Endpoint(s)** |
|---|---|
| **Primary objective(s)** | **Endpoint(s) for primary objective(s)** |
| 1L AML subjects | 1L AML subjects |
| To determine the recommended dose for expansion (RDE) of HDM201 combined with chemotherapy (CT) | Incidence of DLT and time to DLT in subjects receiving induction therapy |
| | Incidence and severity of AEs in 1L AML subjects |
| R/R AML subjects | R/R AML subjects |
| To determine the recommended dose for expansion (RDE) of HDM201 combined with IDAC (cytarabine) | Incidence of DLT and time to DLT |
| | Incidence and severity of AEs in R/R AML subjects |

| **Secondary objective(s)** | **Endpoint(s) for secondary objective(s)** |
|---|---|
| 1L AML and R/R AML subjects | 1L AML and R/R AML subjects |
| To assess PK of HDM201 combined with CT | PK parameters of HDM201 (eg, AUC, Cmax, Tmax) |
| To assess safety and tolerability of HDM201 combined with CT | Incidence of AEs, SAEs and abnormal laboratory values, ECG and vital signs |

| Exploratory objective(s) | Endpoint(s) for exploratory objective(s) |
|---|---|
| 1L AML and R/R AML subjects | 1L AML and R/R AML subjects |
| To assess the relationship between HDM201 systemic exposure (PK) and measures of clinical efficacy, biologic activity and toxicity in combination with CT | Plasma concentration of HDM201/PK-derived metrics, change from baseline in readouts of efficacy (eg, PD markers, peripheral and bone marrow blast count) and safety (eg, platelet count) |
| To assess preliminary efficacy of HDM201 in combination with CT | Proportion of subjects with CR/CRi with adequate blood count recovery (ABCR) |
| | Proportion of subjects receiving HSCT (hematopoietic stem cell transplantation) |
| To assess the blood count recovery of HDM201 in combination with CT | Time to platelet recovery, Time to neutrophil recovery |
| To explore soluble markers relevant to AML and/or to HDM201 mechanism of action (such as GDF15) | Baseline levels and changes from baseline of soluble markers (such as GDF15) and their relationship with PK parameters and response |
| To evaluate the relationship between treatment outcome and genomic and transcriptomic profile (including TP53 mutation or MDM2 expression) | TP53-mutation status, gene expression and mutation profile at baseline and changes from baseline (including CR and relapse) and correlation with treatment response and resistance |

**Table 2-1 Part 2 - Dose Expansion: Objectives and Endpoints**

| **Objective(s)** | **Endpoint(s)** |
|---|---|
| **Primary objective(s)** | **Endpoint(s) for primary objective(s)** |
| 1L AML subjects | 1L AML subjects |
| Expansion Cohort 1 - *de novo* AML subjects without a documented FLT3 mutation: To assess whether RDE of HDM201 in combination with CT is the recommended phase 3 dose (RP3D) | Incidence and severity of AEs, SAEs and abnormal laboratory values, ECG and vital signs |
| | Proportion of CR/CRi with ABCR at the end of induction treatment |
| Expansion Cohort 2 - *de novo* AML subjects with a documented FLT3 mutation: To assess the safety and tolerability of the RDE of HDM201 in combination with 7+3 plus midostaurin | Incidence and severity of AEs, SAEs and abnormal laboratory values, ECG and vital signs |
| Expansion Cohort 3 - secondary AML subjects irrespective of FLT3 mutation: To assess the safety and tolerability of the RDE of HDM201 in combination with liposomal cytarabine/daunorubicin | Incidence and severity of AEs, SAEs and abnormal laboratory values, ECG and vital signs |
| R/R AML subjects | R/R AML subjects |
| Expansion Cohort 4 - R/R AML subjects irrespective of FLT3 mutation: To assess whether RDE of HDM201 in combination with IDAC is the recommended phase 3 dose | Incidence and severity of AEs, SAEs and abnormal laboratory values, ECG and vital signs |
| | Proportion of CR/CRi with ABCR at the end of treatment |

| Secondary objectives | Endpoints for secondary objectives |
|---|---|
| Expansion Cohort 1 - *de novo* AML subjects without a documented FLT3 mutation | Expansion Cohort 1 |
| To assess preliminary efficacy of HDM201 in combination with CT | OS, DFS, CIR , EFS (overall survival, disease free survival, cumulative incidence of relapse, event-free survival) |
| | Proportion of subjects receiving HSCT |

| **Objective(s)** | **Endpoint(s)** |
|---|---|
| To determine the rate of MRD negativity by treatment period | Proportion of subjects with MRD negativity |
| Expansion Cohort 2 - *de novo* AML subjects with a documented FLT3 mutation | Expansion Cohort 2 |
| To assess preliminary efficacy of HDM201 at RDE in combination with CT plus midostaurin | OS, DFS, CIR , EFS, proportion of subjects with CR/CRi with ABCR |
| | Proportion of subjects receiving HSCT |
| To determine the rate of MRD negativity by treatment period | Proportion of subjects with MRD (minimal residual disease) negativity |
| To assess PK of midostaurin in combination with CT and HDM201 at RDE | PK parameters of midostaurin (eg, AUC, Cmax, Tmax) |
| Expansion Cohort 3 - secondary AML subjects irrespective of FLT3 mutation | Expansion Cohort 3 |
| To assess preliminary efficacy of HDM201 at RDE in combination with liposomal cytarabine/daunorubicin | OS, DFS, CIR , EFS, proportion of CR/CRi (complete remission/ Complete Remission with Incomplete Blood Count Recovery) with ABCR (adequate blood count recovery) |
| | Proportion of subjects receiving HSCT |
| Expansion Cohort 4 - R/R AML subjects irrespective of FLT3 mutation | Expansion Cohort 4 |
| To assess preliminary efficacy of HDM201 at RDE in combination with IDAC | OS, EFS |
| | Proportion of subjects receiving HSCT |
| All Expansion Cohorts | All Expansion Cohorts |
| To assess PK of HDM201 at RDE in combination with CT | PK parameters of HDM201 (eg, AUC, Cmax, Tmax) |
| To assess the blood count recovery of HDM201 at RDE in combination with CT | Time to platelet recovery, time to neutrophil recovery |
| To assess the depth of MRD negativity using cellular and molecular methods to predict treatment outcome | Percentage of subjects with MRD-negative status at each treatment period and during follow-up and using different methodologies |
| | (MFC or molecular methods), and correlation of MRD-status and clinical efficacy measures (eg, EFS, OS) |
| Expansion Cohorts 1 and 3 only: | Expansion Cohorts 1 and 3 only: |
| To explore symptom burden, subject experience of disease and treatment-related fatigue, key bothersome symptoms | Changes from baseline in FACIT-Fatigue score. Symptoms reported during baseline and EOT(end of treatment) qualitative interview |

### Rationale for dose/regimen and duration of treatment

The starting dose and regimen selection for HDM201 in dose escalation is based on the previous Phase I dose escalation and expansion study of HDM201 as a single-agent in subjects with R/R AML ([HDM201X2101]). In this Phase I study, HDM201 was investigated in subjects with solid tumors and hematological malignancies. A total of 27 subjects with hematological malignancies (26 AML and one acute lymphoblastic leukemia (ALL)) have been enrolled across various regimens. In these subjects, the most common grade 3/4 AE reported as suspected to study treatment included thrombocytopenia, neutropenia, anemia and Tumor Lysis Syndrome (TLS). According to HDM201X2101, Regimen 2C was determined to be the recommended Phase 2 dose (RP2D) for further development of HDM201 as a single-agent.

Based on the safety findings and signal of activity reported in AML subjects treated with HDM201 according to regimen 2C in HDM201X2101, the present study will further investigate HDM201 in combination with chemotherapies used in AML subjects in both first-line and relapsed/refractory settings. Dose escalation for R/R AML and 1L AML subjects is based on successive increase in the total HDM201 dose either by increasing the daily dose or the number of dosing days. The planned escalation is supported by translational preclinical modeling of tumor bearing rats as well as population PK/PD modeling of platelet (PLT) and BM blast data from Study [HDM201X2101] in R/R AML subjects.

The table in example 1 illustrates various features of HDM201 single-agent activity that have been modeled to choose a HDM201 starting dose, frequency and schedule for the present study.

A starting dose regimen of HDM201 40 mg QD D1-D3 (Dose Cohort 1) for dose escalation has been selected. This corresponds to two dose levels below the expected equivalent of HDM201 single-agent RDE (ie, Regimen 2C as evaluated in [HDM201X2101]: 45 mg QD × 7 days in a 28 days cycle, or 315 mg/cycle), and at this dose level, 35% of subjects are predicted to achieve preclinical derived average target efficacious concentrations per cycle (See Table 4-2), corresponding to a mean sustained reduction of clinical BM blasts in bone marrow for approximately 16.8 days from baseline and limited target myelosuppression. It is expected that HDM201 at 40 mg QD D1-D3 (Dose Level 1) in combination with chemotherapy will be safe. The dose and schedule in subsequent cohorts will be modified to increase the overall dose per cycle.

As the clinical PK/PD safety model predicts potential cumulative HDM201-related safety effects (eg, thrombocytopenia) after 2 or 3 cycles for subjects receiving ≥200 mg/cycle, the study will maintain the per cycle dose for consolidation cycles at 200 mg maximum (ie, 40 mg QD D1 to D5), whereas induction cycle doses may be escalated above 200 mg per cycle (>40 mg QD D1 to D5).

### Population

Two AML populations will be evaluated in this study.

The first population will be 1L AML which includes *de novo* AML subjects (ie, untreated newly diagnosed) and secondary AML subjects deemed by the investigator suitable for treatment with chemotherapy (fit):
- *De novo* AML subjects will be enrolled in Part 1 and in Part 2 Expansion Cohort 1 and 2.
- Secondary AML subjects will only be enrolled in Part 2 Expansion Cohort 3 of this study.

The second population will be R/R AML subjects deemed by the investigator suitable for treatment with IDAC. R/R AML subjects will be enrolled in Part 1, in Part 2 Expansion Cohort 4 and in Part 3.

Both 1L AML and R/R AML subjects will be ≥ 18 years of age.

### Inclusion criteria

Subjects eligible for inclusion in this study must meet all of the following criteria:
All subjects
1. Signed informed consent must be obtained prior to participation in the study.
2. Age ≥ 18 years at the date of signing the informed consent form (ICF).
3. Diagnosis of AML based on WHO 2016 classification (Arber et al 2016). Patients with APL (acute promyelocytic leukemia) with PML-RARA are not eligible.
4. Eastern Cooperative Oncology Group (ECOG) performance status (PS) that is 0 to 2
5. Adequate organ functions:
   - Aspartate aminotransferase (AST) and alanine aminotransferase (ALT) ≤ 3 × upper limit of normal (ULN)
   - Total bilirubin (TBL) ≤ 1.5 × ULN (except in the setting of isolated Gilbert syndrome)
   - Glomerular Filtration Rate (estimation based on Cockcroft-Gault formula) ≥ 30 mL/min
6. Left ventricular ejection fraction (LVEF) > 45%

### Additional inclusion criteria for R/R AML subjects

R/R AML subjects eligible for inclusion in this study must additionally meet the following criteria:
7. Diagnosis of relapsed or refractory AML
8. Suitable for treatment with intermediate dose cytarabine (IDAC) as per investigator judgement
9. For Part 3 only: willing and suitable to participate in DDI Cohort 1 or 2

### Additional inclusion criteria for 1L AML subjects:

1L AML subjects eligible for inclusion in this study must additionally meet the following criteria, as applicable to the part/cohort they are to be enrolled in:
For Part 1 or Part 2 Expansion Cohort 1 or Part 2 Expansion Cohort 2 only:
10. Subjects with *de novo* AML
11. Suitable for induction treatment with cytarabine and anthracyclines as per investigator judgement

For Part 2 Expansion Cohort 2 only:
12. Documented presence of FLT3 mutation (ITD or TKD)
13. Suitable for midostaurin treatment as per investigator judgement

For Part 3 Expansion Cohort 3 only:
14. Subjects with secondary AML (eg, AML with Myelodysplasia-Related Changes/AML-MRC, AML secondary to myelodysplasia/MDS or therapy-related AML). Prior use of hypomethylating agents or other therapies with curative intent for treating previous hematological malignancies or therapy-related AML is allowed.
15. Suitable for induction treatment with liposomal cytarabine/daunorubicin as per investigator judgement

### 2. Clinical Trial CHDM201H12101C

A phase lb, multi-arm, open-label, study of HDM201 in combination with MBG453 or venetoclax in adult subjects with acute myeloid leukemia (AML) or high-risk myelodysplastic syndrome (MDS)

### Rationale and design for dose/regimen and duration of treatment of HDM201 in combination with venetoclax

This is a phase 1b, multi-arm, open-label study of HDM201 in combination with MBG453 in subjects with AML or high-risk MDS.

For all subjects, TP53wt status must be characterized by, at a minimum, no mutations noted in exons 5, 6, 7 and 8.

Subjects will receive HDM201 in combination with MBG453.

The HDM201 dose may be escalated (see Table Example 2-1 for provisional dose levels to be tested). Based on the potential for cumulative HDM201-related safety effects with repeat dosing, subjects will not receive an HDM201 dose greater than the planned highest dose of 40 mg daily (>200 mg/cycle) from cycle 3 onwards.

Upon the completion of the escalation part, MTD(s) and/or RD(s) of HDM201 in combination with MBG453 in AML and high-risk MDS subjects will be determined.

Study treatment will be administered in 28-day dosing cycles.

Each treatment arm will enroll cohorts of 3 to 6 subjects treated with HDM201+MBG453 until MTD(s) and/or RD(s) and regimen for future use are identified.

Additional cohorts of 1 to 10 subjects may be enrolled at a previously tested and declared safe dose level in one or both indications in order to better understand the safety, tolerability, PK and preliminary activity of study treatments.

In this study, the selection of the dose and regimen is based on the currently available preclinical and clinical safety, efficacy, PK and PK/PD modeling information from the first-in-human clinical trial CHDM201X2101 for HDM201 and clinical data from CMBG453X2101 and CPDR001X2105 trials for MBG453.

Safety and efficacy data from the FIH trial in AML subjects suggest that the once daily dosing of HDM201 from day 1 to day 7 of a 28-day cycle would be interesting to pursue in combination.

With this regimen, the RD has been determined as 45 mg HDM201 in hematological tumors in the CHDM201X2101 study. Furthermore, preclinical PKPD tumor growth inhibition modeling of rat xenograft data, as well as clinical PKPD modeling of tumor growth and bone marrow blast data from solid and hematological tumors, has shown that shortening the administration of HDM201 to 5 consecutive days from this original regimen still leads to relevant anti-tumor activity, as HDM201 efficacy appears to be primarily driven by cumulative exposure per cycle (Meille C, Guerreiro N, Jullion A et al (2017) Optimization of the dose and schedule of an HDM2 inhibitor NVP-HDM201 in a first-in-human Phase I study using a mechanism-based PK/PD model. Proceedings of the American Association for Cancer Research Annual Meeting 2017; 2017 Apr 1-5; Washington, DC. Philadelphia (PA): AACR; Cancer Res 2017;77(13 Suppl):Abstract nr CT154. doi:10.1158/1538-7445.AM2017-CT154).

A dose-escalation approach will be undertaken in order to determine the appropriate dose of HDM201 in combination with MBG453. The starting dose of HDM201 tested in combination with MBG453 will be 20 mg. HDM201 will be administered orally once daily from day 1 to day 5 of a 28 days cycle. The total HDM201 dose per cycle will be 3.15-fold lower than the total dose per cycle using the RD defined with the original 7 days regimen in the CHDM201X2101 study. Thus, HDM201 at a starting dose of 20 mg from day 1 to day 5 on a 28 days cycle is expected to be tolerated.

As the PKPD safety model of thrombocytopenia suggests potential cumulative HDM201-related safety effects (i.e. thrombocytopenia) from cycle 2 onwards for subjects receiving ≥200 mg/cycle, the study will maintain the dose for subsequent cycles at a maximum of 200 mg per cycle (i.e. 40 mg daily from day 1 to day 5), whereas the dose in the first 2 cycles may be escalated above 200 mg per cycle (i.e. >40 mg daily from day 1 to day 5). Refer to Table Example 2-1 for HDM201 provisional dose levels.

The MBG453 single agent RD has been determined as 800 mg Q4W in solid tumor subjects primarily based on PK and PKPD modeling of target (TIM-3) occupancy. MBG453 at the dose level of 800 mg Q4W was predicted to give sustained target occupancy of 90% in tumor in > 90% of subjects. No significant safety signal has been detected at any dose of MBG453 up to 1200 mg Q2W or Q4W in the CMBG453X2101 study. MBG453 single agent is also being evaluated in AML/MDS subjects in the CPDR001X2105 study with Q4W and Q2W regimens.

The RD in AML/MDS has not yet been determined, however it is not expected to be different from solid tumors, based on preliminary PK and safety data. MBG453 at the dose levels of 400 mg Q2W and 800 mg Q4W has been well tolerated in AML/MDS and both are similarly expected to achieve a sustained >90% depletion of TIM-3 as a target requirement for efficacy.

The proposed starting dose and regimen for MBG453 in arm 1 will be 400 mg Q2W. However, if emerging data from ongoing CPDR001X2105 study suggest an alternative regimen, switch to 800 mg Q4W that is the RD determined in solid tumors could be considered. Only HDM201 will be dose escalated while MBG453 will be administered at a fixed dose of 400 mg Q2W. Depending on the final results of the CPDR001X2105 study, the RD of 800 mg MBG453 Q4W determined in solid tumor subjects may also be explored.

Based on these prior safety data and the assumptions for DDI, the starting dose for the combination satisfies the EWOC criteria within the BHLRM.

### Rationale for choice of combination drugs

The rationale for combining HDM201 and MBG453 is based on the following evidence:
Primary leukemic blasts overexpress TIM-3 and TIM-3 is modulated upon MDM2 inhibition in both ex vivo human PBMCs and subject samples treated with MDM2 inhibitors.

Preclinical evidence shows that the concurrent blockade of MDM2 and TIM-3 in syngeneic mouse models enhances anti-tumor response.

### Population

The study is conducted in TP53wt adult patients with:
- R/R AML who have failed ≥1 prior regimen, or
- First line AML unfit for standard induction chemotherapy, or
- High-risk MDS who have failed hypomethylating agent therapy.

Only patients who meet all the following inclusion and none of the exclusion criteria are treated in the study. National Cancer Institute CTCAE version 5.0 is used for all grading.

### Inclusion criteria

Patients eligible for inclusion in this study must meet all of the following criteria:
1. Male or female patients ≥ 18 years of age at the date of signing the informed consent form who present with one of the following:
   a. Relapsed/refractory AML following ≥1 prior therapies (but ≤3 prior therapies) who have relapsed or exhibited refractory disease (primary failure) and are deemed by the Investigator not to be candidates for standard therapy, including re-induction withcytarabine or other established chemotherapy regimens for patients with AML (patients who are suitable for standard re-induction chemotherapy or hematopoietic stem cell transplantation and willing to receive it are excluded)
   b. First line AML patient unfit for standard induction chemotherapy (includes both de novo and secondary AML).
   c. High-risk MDS patient (high and very high-risk groups according to rIPSS) who havefailed hypomethylating agent therapy.
2. Eastern Cooperative Oncology Group (ECOG) performance status ≤ 1
3. Tumor of the patient is TP53wt . At minimum exons 5, 6, 7 and 8 in the TP53 gene must be sequenced and determined to contain no mutations. The TP53 status must be obtained from a bone-marrow sample, collected no longer than 3 months before signing the main ICF.
4. Patients are candidates for serial bone marrow aspirate and/or biopsy according to the institutions guidelines and undergo a bone marrow aspirate and/or biopsy at screening, during and at the end of therapy on this study.

### Principle exclusion criteria:

Patients eligible for this study must not meet any of the following criteria:
- Prior combination treatment with compounds having the same mode of action:
   - mdm2 or mdm4 inhibitors combined with TIM-3 inhibitors (for patients enrolled in treatment arm1)
   - mdm2 or mdm4 inhibitors combined with Bcl-2 inhibitor (for patients enrolled in treatment arm2)
- History of severe hypersensitivity reactions to any ingredient of study drug(s) and other monoclonal antibodies (mAbs) and/or their excipients.
- Patients with acute promyelocytic leukemia with PML-RARA.
- Allogeneic stem cell transplant (HSCT) within last 6 months and/or active GvHD requiring systemic immunosuppressive therapy.
- GI disorders impacting absorption of oral HDM201 or venetoclax.
- Evidence of active bleeding or bleeding diathesis or major coagulopathy (including familial).
- Patients with active, known or suspected autoimmune disease(for patients enrolled in treatment arm1).

### Treatment and study drugs

For this study, the term "investigational drug" or "study drug" refers to HDM201 or MBG453. "Treatment arm" or "study treatment" refers to a specific combination treatment i.e. HDM201+MBG453. The investigational drugs used in this study are:
HDM201: 10mg, 20mg, 40mg, Capsule for oral use, 20 mg (starting dose), Day 1 to day 5 (28-day cycle), Open label patient specific; bottles.

MBG453: 100mg/ml LlVI, (Liquid In Vial), Concentrate for Solution for infusion; Intravenous use, 400 mg Once every 2 weeks (Day 1, 15 of 28-day cycle) OR 800 mg Once every 4 (Day 1 of 28-day cycle)weeks; Open label bulk, supply; vials.

No randomization will be performed in this study.

HDM201 capsules will be administered orally (p.o.) in the fasted state at least 1 hour before or 2 hours after a meal. The subject should take the capsules in the morning, at approximately the same time each day of dosing, with a glass of water and without chewing the capsules. If the subject is assigned to a dose level where multiple capsules are to be taken, the capsules should be taken consecutively, within as short an interval as possible. If the subject forgets to take his/her daily dose, then he/she should restart the dose on the next scheduled dosing day without compensating for missed doses. HDM201 is to be administered first.

MBG453 will be administered via i.v. infusion over 30 minutes (up to 2 hours, if clinically indicated) as described in the pharmacy manual starting approximately within the next hour after HDM201 administration, when administered.

A subject may continue study treatment until the subject experiences unacceptable toxicity, disease progression (Cheson BD, Bennett JM, Kopecky K, et al (2003) Revised recommendations of the International Working Group (IWG) for diagnosis, standardization of response criteria, treatment outcomes, and reporting standards for therapeutic trials in acute myeloid leukemia. J Clin Oncol; 21(24):4642-9 and Cheson BD, Greenberg P, Bennett J, et al (2006) Clinical application and proposal for modification of the International Working Group (OWG) response criteria in myelodysplasia. Blood; 108:419-425). If more than 2 consecutive cycles of HDM201+venetoclax have to be skipped due to drug-related toxicities, then the combination of drugs should be permanently discontinued.

### Dose escalation and dose modification

### Starting dose

The starting dose and regimen selection for HDM201 in dose escalation is based on the previous Phase I dose escalation and expansion study of HDM201 as a single-agent in subjects with AML/MDS (CHDM201X2101) in which a dose of 45 mg/day (day 1-7 / 28-day cycle) was determined to be the RD. In this study, a starting dose and regimen of 20 mg/day HDM201 (day 1-5 / 28-day cycle) for dose escalation has been selected. The selection of dose and regimen was supported by single agent translational preclinical modeling of tumor bearing rats and population PK/PD modeling of thrombocytopenia and bone marrow blast data from CHDM201X2101 study in AML/MDS subjects. The starting dose corresponds to -315% below the cumulative dose of HDM201 single agent RD (as evaluated in CHDM201X2101 at 45 mg/day (day 1-7 / 28-day cycle), or 315 mg/cycle). At this dose level, -15% of subjects are predicted to achieve preclinical derived average target efficacious concentrations of HDM201 per cycle, with some anticipated clinical activity (bone marrow blast reduction) and limited target myelosuppression. Additionally, PBPK SimCyp modeling at the 20 mg HDM201 starting dose predicted -1.20-fold increase of venetoclax exposure during the initial ramp-up phase and at steady state.

In the HDM201+MBG453 treatment arm 1, the starting doses for HDM201 and MBG453 are 20 mg/day (day 1-5 / 28-day cycle) and 400 mg (Q2W, 28-day cycle), respectively.

Depending on the results of the ongoing CPDR001X2105 study, MBG453 at 800 mg Q4W may be also explored. Only HDM201 will be dose escalated while MBG453 will be administered at a fixed dose and in a given regimen, i.e. either 400 mg Q2W or 800 mg Q4W. Should an alternative regimen be explored or added (e.g. MBG453 Q4W), dose-DLT data available from the ongoing regimen (e.g. MBG453 Q2W) will be included to derive the starting dose of the new regimen using BHLRM and should be EWOC satisfied.

### Provisional dose levels

The following Table Example 2-1 describes the starting dose and the dose regimen of HDM201 that may be evaluated during the combination HDM201+venetoclax. (1 cycle = 28 days).

### Example 2-1

| Dose level | HDM201 dose, cycles 1-2* | HDM201 dose, cycles ≥3* |
|---|---|---|
| -1 ** | 10 mg, d1-5 | 10 mg, d1-5 |
| 1 (start) | 20 mg, d1-5 | 20 mg, d1-5 |
| 2 | 30 mg, d1-5 | 30 mg, d1-5 |
| 3 | 40 mg, d1-5 | 40 mg, d1-5 |
| 4 | 50 mg, d1-5 | 40 mg, d1-5 |
| 5 | 60 mg, d1-5 | 40 mg, d1-5 |

| | | |
|---|---|---|
| * It is possible for additional and/or intermediate dose levels to be added during the course of the study. Cohorts may be added at any dose level below the MTD in order to better characterize safety, PK or PD. ** Dose level -1 represents treatment dose when dose de-escalation from the starting dose level is required. No dose de-escalation below dose level -1 is permitted for this study. | | |

The following Tables describe the starting dose and the dose regimen of MBG453 that may be evaluated during the HDM201+MBG453 combination (treatment arm 1) for Q2W and Q4W regimen over 28-day cycles.

| Dose level | MBG453 daily dose | MBG 453 dosing frequency |
|---|---|---|
| 1 (start)* | 400 mg | Q2W |

| | | |
|---|---|---|
| * If safety issue is observed at the starting dose, the next cohort will be open at 400 mg Q4W and could be further escalated according to the following table. | | |

| Dose level | MBG453 daily dose | MBG 453 dosing frequency |
|---|---|---|
| 1 (start)* | 800 mg | Q4W |

| | | |
|---|---|---|
| * If safety issue is observed at the starting dose, the next cohort will be open at 400 mg Q4W. | | |

### Objectives and endpoints

| Objectives | Endpoints | |
|---|---|---|
| Primary Objective(s): | Endpoint(s) for primary objective(s) | |
| To characterize safety and tolerability of | Safety: | |
| each treatment arm and identify | - Incidence and severity of AEs and SAEs, including changes in laboratory values, vital signs, and ECGs. | |
| recommended doses and regimens for future | | |
| studies | - Incidence and nature of DLTs. | |
| | Tolerability: Dose interruptions, reductions, and dose intensity | |
| Secondary Objective(s): | Endpoint(s) for secondary objective(s): | |
| To characterize the pharmacokinetic profile of investigational drugs (HDM201 and MBG453) administered in combination. | PK parameters (e.g., AUC, Cmax, Tmax) and concentration vs. time profiles of each investigational drug within combination regimens. | |
| To assess emergence of anti-MBG453 antibodies following one or more i.v. infusions of MBG453 in combination with HDM201 | | |
| | Presence and/or concentration of anti-MBG453 antibodies | |
| To evaluate preliminary anti-tumor activity. | ORR, BOR and: | |
| To assess the pharmacodynamics (PD) effect. | | - EFS, RFS and DOR for AML (Cheson 2003) |
| | | - PFS, TTR and DOR for MDS (Cheson 2006) |
| | Changes from baseline in GDF-15, soluble TIM-3 | |

In the same way the combination HDM201 and venetoclax is studied. In this case, the following is provided:
Venetoclax film-coated tablets will be administered orally once a day according to local label recommendations. Subjects should be instructed to swallow the tablets as a whole with water at approximately the same time each day. The tablets should be taken with a meal (ideally during breakfast) in order to avoid a risk for lack of efficacy.

During the ramp-up period, venetoclax should be taken in the morning to facilitate laboratory monitoring.

If a subject misses a dose of venetoclax within 8 hours of the time it is usually taken, the subject should take the missed dose as soon as possible on the same day. If a subject misses a dose by more than 8 hours, the subject should not take the missed dose and should resume the usual dosing schedule at the usual time the following day. However, in case the missed dose occurs during the ramp-up period of venetoclax, the subject should resume on the following day at the dose that was missed in order not to skip any dose increase of venetoclax during the ramp-up.

A subject may continue study treatment until the subject experiences unacceptable toxicity, disease progression (Cheson et al. 2003 for AML and Cheson et al. 2006 for MDS). If more than 2 consecutive cycles of HDM201+venetoclax have to be skipped due to drug-related toxicities, then the combination of drugs should be permanently discontinued.

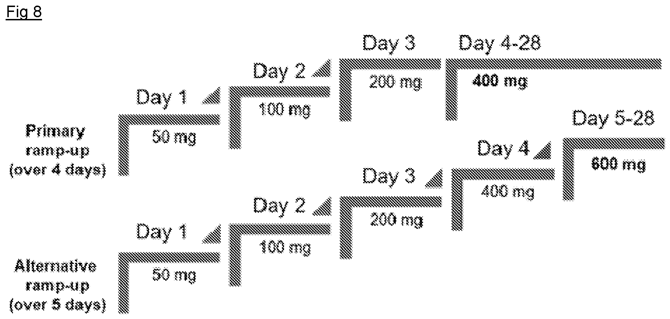

In the HDM201+venetoclax treatment arm 2, the starting dose for HDM201 is 20 mg/day (day 1-5 / 28-day cycle). The venetoclax starting dose of 50 mg will be gradually increased over a period of 4 days as shown in Figure 8 in order to reach the planned target dose of 400 mg/day of venetoclax. Once this target dose is reached, subjects will continue on that dose of venetoclax (28-day cycle). Only one of the two investigational drugs (i.e. HDM201 or venetoclax) can be escalated at a time. However, the escalation of each of the two study drugs may be tested in parallel in two different cohorts but with a reduced cohort size, if both escalations are allowed by the BHLRM (i.e. the corresponding dose combinations are admissible).

The following Table describes the starting dose and ramp-up of venetoclax that may be evaluated during the HDM201+venetoclax combination (treatment arm 2).

| Dose level | Venetoclax daily dose | Venetoclax dosing frequency |
|---|---|---|
| -1* | 300 mg | |
| 1 (start) | 400 mg | Ramp-up 4 days, then daily |
| 2 | 600 mg | Ramp-up 5 days, then daily |

| | | |
|---|---|---|
| * Dose level -1 represents treatment dose when dose de-escalation from the starting dose level is required. No dose de-escalation below dose level -1 is permitted for this study. | | |

### List of abbreviations:

- AE: Adverse Event
- SAE: Serious Adverse Event
- AUC: Area Under the Curve
- AML: Acute Myeloid Leukemia
- R/R: Relapsed/Refractory
- BHLRM: Bayesian Hierarchical Logistic Regression Model
- BM: Bone Marrow
- CR: Complete Remission
- CTCAE: Common Terminology Criteria for Adverse Events
- MDS: Myelodysplastic Syndrome
- MTD: Maximum Tolerated Dose
- RD: Recommended Dose
- FIH: First in Human
- EWOC: Escalation with Overdose Control
- Q4W: Every 4 weeks
- Q2W: Every 2 weeks
- TP53: Tumor Protein 53
- Wt: wild type
- PML-RARA: Promyelocytic leukemia/retinoic acid receptor alpha
- GvHD: Graft versus host disease
- G!: Gastrointestinal
- ECG: Electrocardiogram
- DLT: Dose Limiting Toxicity
- ORR: Overall Response Rate
- BOR: Best Overall Response
- PFS: Progression Free Survival
- TTR: Time To Response
- DOR: Duration of Response
- rIPSS: revised International Prognostic Scoring System
- MFC: Multi-parameter flow cytometry

## Claims

1. The HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one (HDM201) or a pharmaceutically acceptable salt, solvate, hydrate, complex or co-crystal thereof
for use in the treatment of hematological tumors,
wherein the drug is administered on each of the first 3 to 7 days of a 28 days treatment cycle,
wherein the treatment is composed of at least three 28 days treatment cycles,
wherein the daily drug dose for the first and second treatment cycles, which are induction cycles is from 50 mg to 100 mg and the daily drug dose for the third and any following treatment cycles, which are consolidation cycles is from 10 mg to 45 mg.

2. The HDM2-p53 interaction inhibitor drug HDM201, or a pharmaceutically acceptable salt, solvate, hydrate, complex or co-crystal thereof, for use in the treatment of hematological tumors according to claim 1, wherein the drug is administered on each of the first 4 to 6 days, preferably on the first 5 days, of a 28 days treatment cycle.

3. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the daily drug dose of the induction cycles is from 50 mg to 80 mg, preferably from 60 mg to 80 mg, more preferably 60 mg.

4. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the daily drug dose of the consolidation cycles is from 20 mg to 40 mg, preferably from 30 mg to 40 mg, more preferably 40 mg.

5. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the drug is administered on each of the first 5 days of a 28 days treatment cycle, wherein the daily drug dose of the induction cycles is from from 60 mg to 80 mg, and wherein the daily drug dose of the consolidation cycles is 40 mg.

6. The HDM2-p53 interaction inhibitor drug HDM201, or a pharmaceutically acceptable salt, solvate, hydrate, complex or co-crystal thereof, for use in the treatment of hematological tumors according to any one of preceding claims, wherein the hematological tumor is a leukemia.

7. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the hematological tumor is acute myeloid leukemia (AML), preferably relapsed/refractory AML, first line (1L) AML (includes both *de novo* and secondary AML).

8. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the hematological tumor is myelodysplastic syndrome (MDS), preferably high-risk MDS (including high and very high-risk MDS according to rIPSS (revised international prognostic scoring system)).

9. The HDM2-p53 interaction inhibitor drug HDM201, or a pharmaceutically acceptable salt, solvate, hydrate, complex or co-crystal thereof, for use in the treatment of hematological tumors according to any one of preceding claims, wherein the hematological tumor is a TP53 wild-type hematological tumor.

10. The HDM2-p53 interaction inhibitor drug HDM201, or a pharmaceutically acceptable salt, solvate, hydrate, complex or co-crystal thereof, for use in the treatment of hematological tumors according to any one of preceding claims, wherein the drug is present as a salt, solvate, hydrate, complex or co-crystal, more preferably the salt, solvate, hydrate, complex or co-crystal is a co-crystal, even more preferably present as succinic acid co-crystal.

11. The HDM2-p53 interaction inhibitor drug HDM201, or a pharmaceutically acceptable salt, solvate, hydrate, complex or co-crystal thereof, for use in the treatment of hematological tumors according to claim 10, wherein the drug is present as succinic acid co-crystal, preferably as 1:1 molar ratio succinic acid : HDM201 co-crystal.

12. The HDM2-p53 interaction inhibitor drug HDM201, or a pharmaceutically acceptable salt, solvate, hydrate, complex or co-crystal thereof, for use in the treatment of hematological tumors according to any one of preceding claims, wherein the HDM201 drug is combined with one or more other anti-cancer agents, preferably said anti-cancer agent(s) is(are) selected from: immuno-oncological drugs (e.g. PD-1 [e.g. PDR001 (Novartis, INN Spartalizumab)], PD-L1, LAG-3, TIM-3 [e.g. MBG453(Novartis)], GTIR, TGF-beta, IL15 inhibitors), FLT3 inhibitors (e.g. gilterinib, quizartinib, midostaurin), BCL2 inhibitors (e.g. navitoclax, venetoclax), other HDM2 inhibitors (e.g. idasanutlin, AMG232, DS-3032B, ALRN6924/ATSP7041), hypomethylating agents (HMA) (e.g. Vidaza [azacytidine, 5-azacytidine], Dacogen [decitabine], guadecitabine), anthracyclines (e.g. idarubicin, daunorubicin, doxorubicin, epirubicin, rubidomycin); anti-CD33 antibodies (e.g. Mylotarg [gemtuzumab], vadastuximab) and AraC [cytarabine, aracytine]).

13. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the drug HDM201 is combined with one or more therapeutically active agents selected from cytarabine (Ara-C), anthracycline, daunorubicin, idarubicin, rubidomycin, idamycin, midostaurin and azacytidine.

14. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the drug HDM201 is combined with a BCL2 inhibitor, preferably venetoclax.

15. The HDM2-p53 interaction inhibitor drug HDM201, or a pharmaceutically acceptable salt, solvate, hydrate, complex or co-crystal thereof, for use in the treatment of hematological tumors according to any one of the preceding claims, wherein the drug HDM201 is combined with the TIM-3 inhibitor MBG453 (Novartis).

## Patentansprüche

1. HDM2-p53-Wechselwirkungsinhibitor-Arzneistoff (S)-5-(5-Chlor-1-methyl-2-oxo-1,2-dihydropyridin-3-yl)-6-(4-chlorphenyl)-2-(2,4-dimethoxypyrimidin-5-yl)-1-isopropyl-5,6-dihydro-1H-pyrrolo[3,4-d]imidazol-4-on (HDM201) oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon
zur Verwendung bei der Behandlung von hämatologischen Tumoren,
wobei der Arzneistoff an jedem der ersten 3 bis 7 Tage eines 28-tägigen Behandlungszyklus verabreicht wird,
wobei die Behandlung aus mindestens drei 28-tägigen Behandlungszyklen besteht,
wobei die tägliche Arzneistoffdosis für den ersten und den zweiten Behandlungszyklus, bei denen es sich um Induktionszyklen handelt, 50 mg bis 100 mg beträgt und die tägliche Arzneistoffdosis für den dritten und jeden folgenden Behandlungszyklus, bei denen es sich um Konsolidierungszyklen handelt, 10 mg bis 45 mg beträgt.

2. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach Anspruch 1, wobei der Arzneistoff an jedem der ersten 4 bis 6 Tage, vorzugsweise an den ersten 5 Tagen, eines 28-tägigen Behandlungszyklus verabreicht wird.

3. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei die tägliche Arzneistoffdosis der Induktionszyklen 50 mg bis 80 mg, vorzugsweise 60 mg bis 80 mg, weiter bevorzugt 60 mg, beträgt.

4. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei die tägliche Arzneistoffdosis der Konsolidierungszyklen 20 mg bis 40 mg, vorzugsweise 30 mg bis 40 mg, weiter bevorzugt 40 mg, beträgt.

5. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der Arzneistoff an jedem der ersten fünf Tage eines 28-tägigen Behandlungszyklus verabreicht wird, wobei die tägliche Arzneistoffdosis der Induktionszyklen 60 mg bis 80 mg beträgt und wobei die tägliche Arzneistoffdosis der Konsolidierungszyklen 40 mg beträgt.

6. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei es sich bei dem hämatologischen Tumor um eine Leukämie handelt.

7. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei es sich bei dem hämatologischen Tumor um akute myeloische Leukämie (AML), vorzugsweise rezidive/refraktäre AML, Erstlinien(1L)-AML (schließt sowohl de-novo-AML als auch sekundäre AML ein), handelt.

8. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei es sich bei dem hämatologischen Tumor um myelodysplastisches Syndrom (MDS), vorzugsweise MDS mit hohem Risiko (schließt sowohl MDS mit hohem Risiko als auch MDS mit sehr hohem Risiko gemäß dem rIPSS (revised International Prognostic Scoring System) ein), handelt.

9. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei es sich bei dem hämatologischen Tumor um einen hämatologischen TP53-Wildtyp-Tumor handelt.

10. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der Arzneistoff als Salz, Solvat, Hydrat, Komplex oder Cokristall vorliegt, weiter bevorzugt das Salz, das Solvat, das Hydrat, der Komplex oder der Cokristall ein Cokristall ist, noch weiter bevorzugt als Bernsteinsäure-Cokristall vorliegt.

11. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach Anspruch 10, wobei der Arzneistoff als Bernsteinsäure-Cokristall vorliegt, vorzugsweise als Cokristall mit einem Bernsteinsäure:HDM201-Verhältnis von 1:1.

12. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der HDM201-Arzneistoff mit einem oder mehreren anderen Antikrebsmitteln kombiniert ist, wobei das Antikrebsmittel bzw. die Antikrebsmittel vorzugsweise aus: immunonkologischen Arzneistoffen (z. B. PD-1- [z. B. PDR001 (Novartis, INN Spartalizumab)], PD-L1-, LAG-3-, TIM-3- [z. B. MBG453 (Novartis)], GTIR-, TGF-beta-, IL15-Inhibitoren), FLT3-Inhibitoren (z. B. Gilterinib, Quizartinib, Midostaurin), BCL2-Inhibitoren (z. B. Navitoclax, Venetoclax), anderen HDM2-Inhibitoren (z. B. Idasanutlin, AMG232, DS-3032B, ALRN6924/ATSP7041), Hypomethylierungsmitteln (HMA) (z. B. Vidaza [Azacytidin, 5-Azacytidin], Dacogen [Decitabin], Guadecitabin), Anthracyclinen (z. B. Idarubicin, Daunorubicin, Doxorubicin, Epirubicin, Rubidomycin); Anti-CD33-Antikörpern (z. B. Mylotarg [Gemtuzumab], Vadastuximab) und AraC [Cytarabin, Aracytin]) ausgewählt ist bzw. sind.

13. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der Arzneistoff HDM201 mit einem oder mehreren therapeutischen Wirkstoffen, die aus Cytarabin (Ara-C), Anthracyclin, Daunorubicin, Idarubicin, Rubidomycin, Idamycin, Midostaurin und Azacytidin ausgewählt sind, kombiniert ist.

14. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der Arzneistoff HDM201 mit einem BCL2-Inhibitor, vorzugsweise Venetoclax, kombiniert ist.

15. HDM2-p53-Wechselwirkungsinhibitor-Arzneistoff HDM201 oder ein pharmazeutisch unbedenkliches Salz, ein pharmazeutisch unbedenkliches Solvat, ein pharmazeutisch unbedenkliches Hydrat, ein pharmazeutisch unbedenklicher Komplex oder ein pharmazeutisch unbedenklicher Cokristall davon zur Verwendung bei der Behandlung von hämatologischen Tumoren nach einem der vorhergehenden Ansprüche, wobei der Arzneistoff HDM201 mit dem TIM-3-Inhibitor MBG453 (Novartis) kombiniert ist.

## Revendications

1. Médicament inhibiteur de l'interaction HDM2-p53 (S)-5-(5-Chloro-1-méthyl-2-oxo-1,2-dihydro-pyridin-3-yl)-6-(4-chloro-phényl)-2-(2,4-diméthoxy-pyrimidin-5-yl)-1-isopropyl-5,6-dihydro-1H-pyrrolo[3,4-d]imidazol-4-one (HDM201) ou sel, solvate, hydrate, complexe ou cocristal pharmaceutiquement acceptable correspondant
pour une utilisation dans le traitement de tumeurs hématologiques,
le médicament étant administré à chacun des 3 à 7 premiers jours d'un cycle de traitement de 28 jours,
le traitement étant composé d'au moins trois cycles de traitement de 28 jours,
la dose quotidienne de médicament pour le premier et le deuxième cycle de traitement, qui sont des cycles d'induction, étant de 50 mg à 100 mg et la dose quotidienne de médicament pour le troisième cycle et tout cycle de traitement suivant, qui sont des cycles de consolidation, étant de 10 mg à 45 mg.

2. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon la revendication 1, le médicament étant administré à chacun des 4 à 6 premiers jours, préférablement les 5 premiers jours, d'un cycle de traitement de 28 jours.

3. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la dose quotidienne de médicament des cycles d'induction étant de 50 mg à 80 mg, préférablement de 60 mg à 80 g, plus préférablement de 60 mg.

4. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la dose quotidienne de médicament des cycles de consolidation étant de 20 mg à 40 mg, préférablement de 30 mg à 40 g, plus préférablement de 40 mg.

5. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament étant administré à chacun des 5 premiers jours d'un cycle de traitement de 28 jours, la dose quotidienne de médicament des cycles d'induction étant de 60 mg à 80 mg, et la dose quotidienne de médicament des cycles de consolidation étant de 40 mg.

6. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la tumeur hématologique étant une leucémie.

7. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la tumeur hématologique étant une leucémie myéloïde aiguë (AML), préférablement une AML récidivante/réfractaire, une AML de première ligne (1L) (comprenant à la fois une AML *de novo* et secondaire).

8. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la tumeur hématologique étant un syndrome myélodysplasique (MDS), préférablement un MDS à haut risque (y compris un MDS à haut et très haut risque selon le rIPSS (système de notation de pronostic international révisé).

9. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, la tumeur hématologique étant une tumeur hématologique de type sauvage *TP53.*

10. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament étant présent en tant qu'un sel, solvate, hydrate, complexe ou cocristal, plus préférablement, le sel, le solvate, l'hydrate, le complexe ou le cocristal étant un cocristal, encore plus préférablement présent en tant que cocristal avec l'acide succinique.

11. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon la revendication 10, le médicament étant présent en tant que cocristal avec l'acide succinique préférablement comme cocristal d'acide succinique : HDM201 à un rapport molaire de 1 : 1.

12. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament HDM201 étant combiné avec un ou plusieurs autres agents anticancéreux, préférablement ledit ou lesdits agents cancéreux étant choisis parmi : des médicaments immuno-oncologiques (par ex., PD-1 [par ex., PDR001 (Novartis, INN spartalizumab)], PD-L1, LAG-3, TIM-3 [par ex., MBG453 (Novartis)], GTIR, TGF-bêta, inhibiteurs d'IL15), inhibiteurs de FLT3 (par ex., giltérinib, quizartinib, midostaurine), inhibiteurs de BCL2 (par ex., navitoclax, vénétoclax), autres inhibiteurs de HDM2 (par ex., idasanutline, AMG232, DS-3032B, ALRN6924/ATSP7041), agents hypométhylants (HMA) (par ex., Vidaza [azacytidine, 5-azacytidine], Dacogen [décitabine], guadécitabine), anthracyclines (par ex., idarubicine, daunorubicine, doxorubicine, épirubicine, rubidomycine) anticorps anti-CD33 (par ex., Mylotarg [gémtuzumab], vadastuximab) et AraC [cytarabine, aracytine]).

13. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament HDM201 étant combiné avec un ou plusieurs autres agents thérapeutiquement actifs choisis parmi cytarabine (Ara-C), anthracycline, daunorubicine, idarubicine, rubidomycine, idamycine, midostaurine et azacytidine.

14. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament HDM201 étant combiné avec un inhibiteur de BCL2, préférablement vénétoclax.

15. Médicament inhibiteur de l'interaction HDM2-p53 HDM201, ou sel, solvate, hydrate, complexe ou cocristal pharmaceutiquement acceptable correspondant, pour une utilisation dans le traitement de tumeurs hématologiques selon l'une quelconque des revendications précédentes, le médicament HDM201 étant combiné avec l'inhibiteur de TIM-3 MBG453 (Novartis).
